# FASCICULE DE BREVET EUROPEEN

(11) **EP 4 081 488 B1**
(45) Date de publication et mention de la délivrance du brevet: **11.09.2024**
(21) Numéro de dépôt: 20845409.0
(22) Date de dépôt: 14.12.2020
(51) Int. Cl.: C02F 3/02, C02F 3/12, C12M 1/36, C12Q 1/04, C02F 3/32, C12Q 1/10, C02F 103/00, C02F 3/00

(54) **SYSTÈME ET PROCEDE DE TRAITEMENT DES EAUX GRISES D'UNE HABITATION OU D'UN IMMEUBLE D'HABITATIONS**
SYSTEM UND VERFAHREN ZUM BEHANDELN VON GRAUWASSER EINER WOHNUNG ODER EINES WOHNUNGSBLOCKS
SYSTEM AND PROCESS FOR TREATING THE GREYWATER OF A DWELLING OR OF A BLOCK OF DWELLINGS

(30) Priorité: 24.12.2019 FR 1915553
(43) Date de publication de la demande: 02.11.2022
(73) Titulaire: Ecole Nationale des Ponts et Chaussées, 77420 Champs sur Marne, Marne la Vallée Cedex 2 (FR); Université Paris-Est Créteil Val de Marne, 94010 Creteil Cedex (FR)
(72) Inventeur: MOILLERON, Régis, 91800 BRUNOY (FR); DEROUBAIX, José-Frédéric, 93230 ROMAINVILLE (FR); DUBOIS, Philippe, 77640 JOUARRE (FR)
(74) Mandataire: Brevalex
(86) Numéro de dépôt international: PCT/FR2020/052412
(87) Numéro de publication internationale: WO 2021/130428

(56) Documents cités:
- EP-A2- 0 304 406
- EP-B1- 0 304 406
- DE-U1- 8 623 413
- MA-A1- 20 150 209
- US-A1- 2010 193 413
- US-A1- 2018 057 379

## Description

### DOMAINE TECHNIQUE

L'invention concerne une installation de traitement des eaux grises par phyto-épuration destinée à équiper une habitation ou un immeuble d'habitations, les eaux grises traitées étant réinjectées vers les toilettes et éventuellement les lave-linges.

### ÉTAT DE LA TECHNIQUE ANTÉRIEURE

L'invention concerne le recyclage des eaux grises, qui désignent les eaux issues des lavabos, des douches et des bains, et éventuellement des lave-linge et lave-vaisselles, à la différence des eaux noires qui désignent essentiellement les eaux issues des toilettes.

Les eaux grises étant peu grasses et faiblement polluées, elles peuvent être traitées par phyto-épuration, pour être réinjectées dans l'habitation après épuration, afin d'alimenter des toilettes, et éventuellement des lave-linges.

Le traitement d'eaux grises par phyto-épuration consiste à faire circuler l'eau à épurer dans un filtre composé de plantes spécifiques, de substrats, de microorganismes et autres, ce filtre pouvant s'étendre horizontalement dans un bassin ou bien verticalement sur une paroi appropriée.

D'une manière générale, il est nécessaire que la qualité de l'eau soit contrôlée pour décider si elle peut ou non être fournie à une habitation. Dans le cas des réseaux d'alimentation urbains habituels, ce contrôle se traduit entre autres par des tests bactériologiques réalisés à intervalles réguliers, par exemple une semaine. Le Document EP 0 304 406 A2 divulgue un système comprenant un appareillage de contrôle bactériologique. Les Documents MA 20 150 209 A1 et US 2018/057379 A1 divulguent un système de recyclage d'eau usée.

Dans le cas d'un traitement par phyto-épuration mis en oeuvre à l'échelle d'un immeuble, ce contrôle doit être effectué à une fréquence relativement élevée, pour que de l'eau non conforme ne puisse pas être distribuée dans les habitations. Par exemple en cas de variations brutales de la composition des eaux grises traitées, ou de fluctuation brutale du traitement par phytoépuration, il importe que le système ait une réactivité suffisante pour assurer que l'eau épurée non conforme ne soit pas réinjectée dans l'immeuble.

Néanmoins, un contrôle bactériologique d'un échantillon d'eau prend un temps important, compris entre plusieurs heures et plusieurs jours, selon l'équipement utilisé.

Le but de l'invention est d'apporter une architecture de recyclage d'eaux grises intégrant un contrôle bactériologique capable de fonctionner en continu, en dépit du temps important nécessaire à l'établissement d'un contrôle bactériologique proprement dit.

### EXPOSÉ DE L'INVENTION

A cet effet, l'invention a pour objet un système de traitement d'eaux grises comprenant : une station de phyto-épuration recevant des eaux grises et délivrant de l'eau épurée ; une première et une seconde cuve alimentées sélectivement en eau épurée issue de la station de phyto-épuration via une électrovanne trois-voies, et deux électrovannes pour vider sélectivement leurs contenus vers un réseau d'assainissement ; une cuve principale alimentée par la première et la seconde cuve via respectivement une première et une deuxième électrovanne ; un appareillage de contrôle bactériologique relié à la première et à la seconde cuve pour réaliser sélectivement pour la première ou la seconde cuve un contrôle par analyse bactériologique nécessitant un temps prédéterminé pour son établissement ; et un automate pilotant l'appareillage de contrôle bactériologique et les électrovannes pour commander cycliquement la succession d'opérations suivantes :
- commander l'électrovanne trois-voies pour qu'elle alimente la première cuve et déclencher l'établissement d'un contrôle bactériologique de l'eau épurée contenue dans la seconde cuve ;
- à l'issue du temps d'établissement du contrôle, transférer le contenu de la première cuve vers la cuve principale si le contrôle est favorable ou vers le réseau d'assainissement si le contrôle est défavorable ;
- commander l'électrovanne trois-voies pour qu'elle alimente la seconde cuve et déclencher l'établissement d'un contrôle bactériologique de l'eau épurée contenue dans la première cuve ;
- à l'issue du temps d'établissement du contrôle, transférer le contenu de la seconde cuve vers la cuve principale si le contrôle est favorable ou vers le réseau d'assainissement si le contrôle est défavorable.

Avec cette solution, l'eau épurée peut être traitée et contrôlée de manière continue, de façon à assurer que toute l'eau contenue dans la cuve principale est une eau épurée qui a été effectivement contrôlée favorablement et qui peut être réutilisée.

L'invention a également pour objet un système ainsi défini, comprenant une électrovanne pour vider tout ou partie du contenu de la cuve principale vers le réseau d'assainissement.

L'invention a également pour objet un système ainsi défini, comprenant une cuve supplémentaire destinée à recevoir un trop plein d'eau épurée ne pouvant être contenu dans la cuve principale.

L'invention a également pour objet un système ainsi défini, dans lequel le contrôle bactériologique est défavorable en cas de présence de bactéries d'Escherichia coli ou de coliformes totaux, et favorable sinon.

L'invention a également pour objet un procédé de traitement d'eaux grises dans une installation comprenant : une station de phyto-épuration recevant des eaux grises et délivrant de l'eau épurée ; une première et une seconde cuve alimentées sélectivement en eau épurée issue de la station de phyto-épuration ; une cuve principale alimentée par la première ou la seconde cuve ; un appareillage de contrôle bactériologique relié à la première et à la seconde cuve pour réaliser sélectivement pour la première ou la seconde cuve un contrôle par analyse bactériologique nécessitant un temps prédéterminé pour son établissement ; ce procédé comprenant la succession d'opérations suivantes déclenchée cycliquement :
- alimenter la première cuve avec l'eau épurée de la station de phyto-épuration, et déclencher l'établissement par l'appareillage d'un contrôle bactériologique de l'eau épurée contenue dans la seconde cuve ;
- à l'issue du temps d'établissement du contrôle, transférer le contenu de la première cuve vers la cuve principale si le contrôle est favorable ou vers un réseau d'assainissement si le contrôle est défavorable ;
- alimenter la seconde cuve avec l'eau épurée de la station de phyto-épuration et déclencher l'établissement par l'appareillage d'un contrôle bactériologique de l'eau épurée contenue dans la première cuve ;
- à l'issue du temps d'établissement du contrôle, transférer le contenu de la seconde cuve vers la cuve principale si le contrôle est favorable ou vers le réseau d'assainissement si le contrôle est défavorable.

L'invention concerne également un procédé ainsi défini, dans lequel le contrôle bactériologique est défavorable en cas de présence de bactéries d'Escherichia coli ou de coliformes totaux, et favorable sinon.

### BRÈVE DESCRIPTION DES DESSINS

La Figure 1 est une vue schématique d'ensemble d'un immeuble d'habitation équipé d'une installation de recyclage selon l'invention ;
la Figure 2 est une vue schématique d'ensemble d'une station de contrôle de qualité dans l'installation selon l'invention.

### EXPOSÉ DÉTAILLÉ DE MODES DE RÉALISATION PARTICULIERS

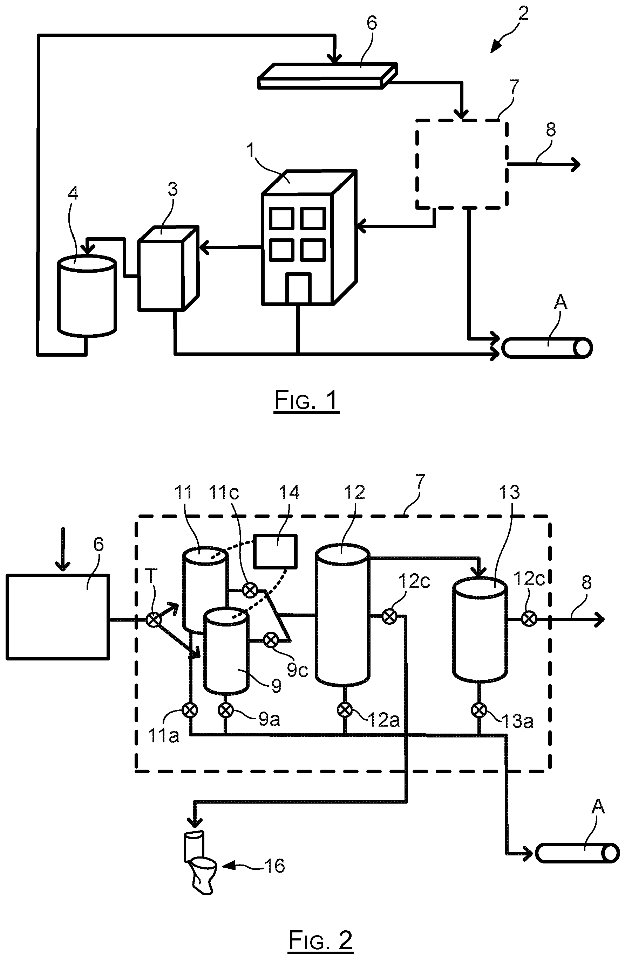

Sur la figure 1, un immeuble d'habitation 1 est équipé d'une installation 2 de recyclage des eaux grises qu'il génère.

Cette installation 2 comporte une cuve 3 de récupération des eaux grises issues de l'immeuble, alimentant en eaux grises une bâche de pompage 4 qui est une cuve équipée d'une pompe pour relever l'eau vers une station de phyto-épuration 6 située en hauteur.

L'eau épurée dans la station 6 est envoyée à une station de contrôle de qualité 7 afin d'être réinjectée dans l'immeuble 1 ou mise à disposition pour alimenter un réseau externe 8 de type irrigation de jardin ou analogue lorsque sa qualité est satisfaisante, ou bien renvoyée vers un réseau d'assainissement A lorsque sa qualité n'est pas satisfaisante.

Comme visible sur le schéma de la figure 1, les eaux dites noires de l'immeuble, c'est-à-dire issues des toilettes ou analogues sont directement renvoyées au réseau d'assainissement A. Par ailleurs, en cas de surplus d'eaux grises ne pouvant être contenues dans la cuve 3 un trop plein permet d'évacuer la surverse vers le réseau d'assainissement A.

La station de phyto-épuration 6 est avantageusement située en toiture de l'immeuble 1, ce qui permet de bénéficier d'un espace suffisant et accessible en milieu urbain, et de collecter également les eaux de pluie pour accroître la quantité d'eau disponible pour l'épuration.

Cette station de phyto-épuration permet d'épurer les eaux grises pour les rendre non pas potables mais utilisables pour alimenter des toilettes. Ce type d'installation, connu en soi, utilise des plantes spécifiques, des substrats et des microorganismes pour constituer un filtre planté, dans un bassin ou analogue, où l'on fait circuler l'eau à épurer, le filtre ainsi constitué pouvant s'étendre horizontalement lorsqu'il s'agit d'un bassin, et/ou verticalement en étant porté par une paroi appropriée. La phyto-épuration est un traitement connu en soi, utilisé généralement au sol, dans le traitement des eaux grises et des eaux noires.

La station de contrôle 7 est alimentée par l'eau issue de la station de phyto-épuration 6. Elle comporte une première cuve de contrôle 9 et une seconde cuve de contrôle 11 de mêmes capacités, aptes l'une et l'autre à alimenter une cuve principale 12 de stockage l'eau épurée pouvant être réinjectée dans l'immeuble. Cette station 7 comprend une cuve supplémentaire 13 destinée à stocker un surplus de la cuve principale 12 afin de le mettre à disposition pour un usage externe tel qu'irrigation, lavage de parkings ou analogue.

La première et la seconde cuve 9 et 11 sont reliées l'une et l'autre à un appareillage de contrôle bactériologique 14 capable de déterminer en quelques heures si l'eau contenue dans l'une ou l'autre de ces cuves peut être consommée ou non, selon qu'elle remplit ou non les critères bactériologiques en vigueur. Cet appareillage 14 établit en quelques heures une analyse bactériologique d'un échantillon de 100 millilitres pour déterminer s'il contient ou non des germes pathogènes d'Escherichia coli ou de coliformes totaux.

Il est nécessaire que ces germes soient absents de l'eau traitée pour qu'elle puisse être réutilisée, c'est-à-dire réinjectée dans l'immeuble pour être utilisée dans les toilettes, et éventuellement pour les lave-linges.

Un appareillage de contrôle bactériologique tel que l'appareillage 14 est d'une manière générale connu en soi. Un appareillage de ce type est vendu sous le nom commercial BACTcontrol, il est par exemple décrit dans le document de brevet EP 2 165 193.

L'appareillage 14 est relié aux cuves 9 et 11 par deux tuyaux équipés d'électrovannes non représentées lui permettant de prélever un échantillon d'eau de l'une ou l'autre de ces cuves pour en faire l'analyse.

Les différentes cuves de la station de contrôle 7 sont reliées les unes aux autres par un ensemble d'électrovannes pilote par un automate non représenté, auquel elles sont reliées. Cet automate est également relié à l'appareillage 14 pour le commander, de façon à piloter l'ensemble de l'installation.

La station de phyto-épuration 6 est ainsi reliée aux cuves 11 et 12 par une électrovanne de type trois-voies repérée par T, permettant d'alimenter en eau épurée soit la cuve 11, soit la cuve 12.

La première cuve de contrôle 9 est reliée à la cuve principale 12 par une électrovanne 9c pour alimenter celle-ci avec de l'eau épurée apte à être consommée, c'est-à-dire contrôlée favorablement par l'appareillage 14, et elle est également reliée au réseau d'assainissement A par l'intermédiaire d'une électrovanne 9a, pour vider son contenu dans le réseau A lorsque l'appareillage 14 a conclu contrôle défavorable c'est-à-dire attestant que son contenu est impropre à la consommation.

La seconde cuve de contrôle 11 est reliée à la cuve principale 12 par une électrovanne 11c pour alimenter celle-ci avec de l'eau épurée apte à la consommation, c'est-à-dire contrôlée favorablement par l'appareillage 14, et elle est également reliée au réseau d'assainissement A par l'intermédiaire d'une électrovanne 11a, pour vider son contenu dans le réseau A lorsque l'appareillage 14 a conclu défavorablement c'est-à-dire par un contrôle attestant que son contenu est impropre à la consommation.

Les électrovannes 9a et 11a peuvent également être utilisées pour vider les cuves 9 et 11 lors d'une opération de maintenance.

La cuve principale 12 est reliée au réseau de l'immeuble alimentant les toilettes 16 et éventuellement les lave-linges par une électrovanne 12c de manière à alimenter l'immeuble en eau épurée réutilisable. Cette cuve principale 12 est aussi reliée à la cuve supplémentaire 13 par une canalisation simple pour transférer son éventuel trop-plein vers la cuve supplémentaire 13. Complémentairement, la cuve 12 est encore reliée au réseau d'assainissement A par une autre électrovanne 12a, ce qui permet par exemple de la vider lors d'une opération de maintenance.

La cuve supplémentaire 13 est quant à elle reliée à un réseau externe 8 par une électrovanne 13c, et elle est également reliée au réseau d'assainissement A par une électrovanne 13a permettant de vider son éventuel trop-plein, et/ou de la vider lors d'une opération de maintenance.

En fonctionnement, les eaux grises générées par les habitations de l'immeuble 1 sont collectées par la bâche 4 et remontées en toiture pour alimenter la station 6 de phyto-épuration. L'électrovanne trois-voies T est alors configurée pour que l'eau épurée issue de la station 6 alimente la première cuve 9. Pendant cette phase, la seconde cuve 11 n'est pas alimentée, mais elle contient de l'eau épurée en cours de contrôle bactériologique par l'appareillage 14.

Lorsque le résultat du contrôle bactériologique est prêt pour la seconde cuve 11, ce qui prend par exemple trois heures, deux possibilités se présentent selon le résultat de ce contrôle.

Si le contrôle est positif, c'est-à-dire si l'eau épurée de la seconde cuve 11 peut être réutilisée, l'électrovanne 11c est commandée en ouverture pour transférer l'eau épurée de cette seconde cuve 11 dans la cuve principale 12.

Si le contrôle est négatif, c'est-à-dire si l'eau épurée de la seconde cuve ne peut pas être réutilisée, l'électrovanne 11a est commandée en ouverture pour transférer l'eau de cette seconde cuve 11 vers le réseau d'assainissement A.

Lorsque le transfert est terminé, l'électrovanne 11c est commandée en fermeture et l'électrovanne trois-voies T est commandée pour que la station 6 alimente la seconde cuve 11. Complémentairement, le système commande l'appareillage 14 pour enclencher un contrôle bactériologique du contenu de la première cuve 9, qui vient d'être alimentée en eau épurée par la station 6.

A ce stade, le processus continue de la même manière, les rôles de la première cuve 9 et de la seconde cuve 11 étant alors intervertis : un contrôle bactériologique est en cours pour la première cuve 9, alors que la seconde cuve 11 est en cours de remplissage.

De manière analogue lorsque le résultat du contrôle de la première cuve 9 est obtenu, l'électrovanne 9c est commandée en ouverture pour transférer son contenu dans la cuve principale 12 si le contrôle est favorable. Sinon, c'est l'électrovanne 9a qui est commandée en ouverture pour transférer le contenu vers le réseau d'assainissement A, dans le cas où le contrôle est défavorable.

Une fois le transfert terminé, l'électrovanne 9c ou 9a qui a été ouverte est commandée en fermeture, puis l'électrovanne trois-voies T est commandée pour que la station 6 alimente la première cuve 9, et l'appareillage 14 est piloté pour débuter un contrôle bactériologique de la seconde cuve 11, qui s'est remplie durant le contrôle de la première cuve 9.

Comme on l'aura compris, l'invention permet d'utiliser deux cuves tampon indépendantes, à savoir les cuves 9 et 11 pilotées pour continuellement collecter l'eau épurée et contrôler son niveau bactériologique avant de transférer cette eau dans une cuve de stockage, à savoir la cuve 12, en vue de la consommation de cette eau dans l'immeuble.

Parallèlement au fonctionnement décrit ci-dessus, lorsque la capacité de la cuve principale 12 est dépassée, alors l'eau épurée excédentaire est transférée vers la cuve supplémentaire 13 pour être utilisée à des fins d'irrigation ou autre. En cas d'excès allant au-delà de la capacité de la cuve supplémentaire 13, l'eau épurée excédentaire peut être évacuée vers le réseau d'assainissement A en actionnant l'électrovanne 12a ou 13a.

Lorsque pour une raison ou une autre, l'eau épurée ne remplit pas les critères bactériologiques, elle est évacuée vers le réseau d'assainissement, la cuve principale n'étant alors plus alimentée tant que la qualité de l'eau épurée n'est pas devenue à nouveau satisfaisante. Dans cette situation, lorsque la cuve principale 12 est complétement vide, l'automate de l'installation pilote des électrovannes appropriées pour que les toilettes et les lave-linges reçoivent de l'eau potable du réseau d'alimentation urbain, de manière à être utilisable sans que les habitants n'aient à intervenir. Ceci peut être obtenu en fermant l'électrovanne 12c située en aval de la cuve principale 12, et en prévoyant une électrovanne additionnelle pouvant être commandée pour alimenter en eau potable la sortie de la cuve principale 12 en aval de l'électrovanne 12c.

Lorsque cette situation est résolue, par exemple par une intervention sur la station 6 de phyto-épuration, les contrôles effectués par l'appareillage 14 redeviennent favorables de sorte que la cuve principale 12 est à nouveau alimentée, ce qui permet à l'automate de réactiver l'alimentation des toilettes et autres de l'immeuble en eau épurée consommable.

## Revendications

1. Système de traitement d'eaux grises comprenant : une station de phyto-épuration (6) recevant des eaux grises et délivrant de l'eau épurée ; une première et une seconde cuve (9, 11) alimentées sélectivement en eau épurée issue de la station de phyto-épuration (6) via une électrovanne trois-voies (T), et deux électrovannes (9a, 11a) pour vider sélectivement leurs contenus vers un réseau d'assainissement (A) ; une cuve principale (12) alimentée par la première et la seconde cuve (9, 11) via respectivement une première et une deuxième électrovanne (9c, 11c) ; un appareillage de contrôle bactériologique (14) relié à la première et à la seconde cuve (9, 11) pour réaliser sélectivement pour la première ou la seconde cuve (9, 11) un contrôle par analyse bactériologique nécessitant un temps prédéterminé pour son établissement ; et un automate pilotant l'appareillage de contrôle bactériologique (14) et les électrovannes (T, 9c, 11c, 9a, 11a) pour commander cycliquement la succession d'opérations suivantes :
- commander l'électrovanne trois-voies (T) pour qu'elle alimente la première cuve (9) et déclencher l'établissement d'un contrôle bactériologique de l'eau épurée contenue dans la seconde cuve (11) ;
- à l'issue du temps d'établissement du contrôle, transférer le contenu de la seconde cuve (11) vers la cuve principale (12) si le contrôle est favorable ou vers le réseau d'assainissement (A) si le contrôle est défavorable ;
- commander l'électrovanne trois-voies (T) pour qu'elle alimente la seconde cuve (11) et déclencher l'établissement d'un contrôle bactériologique de l'eau épurée contenue dans la première cuve (9) ;
- à l'issue du temps d'établissement du contrôle, transférer le contenu de la première cuve (9) vers la cuve principale (12) si le contrôle est favorable ou vers le réseau d'assainissement (A) si le contrôle est défavorable.

2. Système selon la revendication 1, comprenant une électrovanne pour vider tout ou partie du contenu de la cuve principale (12) vers le réseau d'assainissement (A).

3. Système selon la revendication 1 ou 2, comprenant une cuve supplémentaire (13) destinée à recevoir un trop plein d'eau épurée ne pouvant être contenu dans la cuve principale (12).

4. Système selon l'une des revendications précédentes, comportant un appareillage de contrôle dédié à la détection de la présence de bactéries d'Escherichia coli et/ou de coliformes totaux, et dans lequel le contrôle bactériologique est défavorable en cas de présence de bactéries d'Escherichia coli ou de coliformes totaux, et favorable sinon.

5. Procédé de traitement d'eaux grises dans une installation comprenant : une station de phyto-épuration (6) recevant des eaux grises et délivrant de l'eau épurée ; une première et une seconde cuve (9, 11) alimentées sélectivement en eau épurée issue de la station de phyto-épuration (6) ; une cuve principale (12) alimentée par la première ou la seconde cuve (9, 11) ; un appareillage de contrôle bactériologique (14) relié à la première et à la seconde cuve (9, 11) pour réaliser sélectivement pour la première ou la seconde cuve (9, 11) un contrôle par analyse bactériologique nécessitant un temps prédéterminé pour son établissement ; ce procédé comprenant la succession d'opérations suivantes déclenchée cycliquement :
- alimenter la première cuve (9) avec l'eau épurée de la station de phyto-épuration, et déclencher l'établissement par l'appareillage (14) d'un contrôle bactériologique de l'eau épurée contenue dans la seconde cuve (11) ;
- à l'issue du temps d'établissement du contrôle, transférer le contenu de la seconde cuve (11) vers la cuve principale (12) si le contrôle est favorable ou vers un réseau d'assainissement (A) si le contrôle est défavorable ;
- alimenter la seconde cuve (11) avec l'eau épurée de la station de phyto-épuration et déclencher l'établissement par l'appareillage (14) d'un contrôle bactériologique de l'eau épurée contenue dans la première cuve (9) ;
- à l'issue du temps d'établissement du contrôle, transférer le contenu de la première cuve (9) vers la cuve principale (12) si le contrôle est favorable ou vers le réseau d'assainissement (A) si le contrôle est défavorable.

6. Procédé selon la revendication 5, dans lequel le contrôle bactériologique est défavorable en cas de présence de bactéries d'Escherichia coli ou de coliformes totaux, et favorable sinon.

## Patentansprüche

1. System zum Behandeln von Grauwasser, umfassend: eine Pflanzenkläranlage (6), die Grauwasser aufnimmt und geklärtes Wasser abgibt; einen ersten und einen zweiten Behälter (9, 11), die über ein Dreiwege-Magnetventil (T) selektiv mit aus der Pflanzenkläranlage (6) stammendem geklärtem Wasser versorgt werden, und zwei Magnetventile (9a, 11a), um deren Inhalte selektiv in Richtung eines Abwassernetzes (A) zu leeren; einen Hauptbehälter (12), der durch den ersten und den zweiten Behälter (9, 11) über jeweils ein erstes und ein zweites Magnetventil (9c; 11c) versorgt wird; ein Gerät zur bakteriologischen Kontrolle (14), das mit dem ersten und mit dem zweiten Behälter (9, 11) verbunden ist, um selektiv für den ersten oder den zweiten Behälter (9, 11) eine Kontrolle durch bakteriologische Analyse durchzuführen, für deren Erstellung eine vorbestimmte Zeit benötigt wird; und einen Automaten, der das Gerät zur bakteriologischen Kontrolle (14) und die Magnetventile (T, 9c, 11c, 9a, 11a) ansteuert, um zyklisch die Aufeinanderfolge von folgenden Operationen zu steuern:
- Steuern des Dreiwege-Magnetventils (T), damit es den ersten Behälter (9) versorgt, und Auslösen der Erstellung einer bakteriologischen Kontrolle des in dem zweiten Behälter (11) enthaltenen geklärten Wassers;
- am Ende der Zeit zur Erstellung der Kontrolle, Übertragen des Inhalts des zweiten Behälters (11) in Richtung des Hauptbehälters (12), falls die Kontrolle positiv ist, oder in Richtung des Abwassernetzes (A), wenn die Kontrolle negativ ist;
- Steuern des Dreiwege-Magnetventils (T), damit es den zweiten Behälter (11) versorgt und Auslösen der Erstellung einer bakteriologischen Kontrolle des in dem ersten Behälter (9) enthaltenen geklärten Wassers;
- am Ende der Zeit zur Erstellung der Kontrolle, Übertragen des Inhalts des ersten Behälters (9) in Richtung des Hauptbehälters (12), falls die Kontrolle positiv ist, oder in Richtung des Abwassernetzes (A), wenn die Kontrolle negativ ist.

2. System nach Anspruch 1, umfassend ein Magnetventil zum Leeren des gesamten Inhalts des Hauptbehälters (12) oder eines Teils davon in Richtung des Abwassernetzes (A).

3. System nach Anspruch 1 oder 2, umfassend einen zusätzlichen Behälter (13), der dazu bestimmt ist, einen Überlauf an geklärtem Wasser aufzunehmen, das in dem Hauptbehälter (12) nicht enthalten sein kann.

4. System nach einem der vorstehenden Ansprüche, das ein Kontrollgerät beinhaltet, das zum Erkennen des Vorhandenseins von Escheria coli-Bakterien und/oder von gesamtkoliformen Bakterien bestimmt ist, und in dem die bakteriologische Kontrolle im Falle von Vorhandensein von Escheria coli-Bakterien und/oder von gesamtkoliformen Bakterien negativ, und ansonsten positiv ist.

5. Verfahren zum Behandeln von Grauwasser in einer Installation, umfassend: eine Pflanzenkläranlage (6), die Grauwasser aufnimmt und geklärtes Wasser abgibt; einen ersten und einen zweiten Behälter (9, 11), die selektiv mit aus der Pflanzenkläranlage (6) stammendem geklärtem Wasser versorgt werden; einen Hauptbehälter (12), der durch den ersten oder den zweiten Behälter (9, 11) versorgt wird; ein Gerät zur bakteriologischen Kontrolle (14), das mit dem ersten und mit dem zweiten Behälter (9, 11) verbunden ist, um selektiv für den ersten oder den zweiten Behälter (9, 11) eine Kontrolle durch bakteriologische Analyse durchzuführen, für deren Erstellung eine vorbestimmte Zeit benötigt wird; wobei dieses Verfahren die Aufeinanderfolge von folgenden zyklisch ausgelösten Operationen umfasst:
- Versorgen des ersten Behälters (9) mit dem geklärten Wasser aus der Pflanzenkläranlage, und Auslösen der Erstellung durch das Gerät (14) einer bakteriologischen Kontrolle des in dem zweiten Behälter (11) enthaltenen geklärten Wassers;
- am Ende der Zeit zur Erstellung der Kontrolle, Übertragen des Inhalts des zweiten Behälters (11) in Richtung des Hauptbehälters (12), falls die Kontrolle positiv ist, oder in Richtung eines Abwassernetzes (A), wenn die Kontrolle negativ ist;
- Versorgen des zweiten Behälters (11) mit dem geklärten Wasser aus der Pflanzenkläranlage, und Auslösen der Erstellung durch das Gerät (14) einer bakteriologischen Kontrolle des in dem ersten Behälter (9) enthaltenen geklärten Wassers;
- am Ende der Zeit zur Erstellung der Kontrolle, Übertragen des Inhalts des ersten Behälters (9) in Richtung des Hauptbehälters (12), falls die Kontrolle positiv ist, oder in Richtung des Abwassernetzes (A), wenn die Kontrolle negativ ist.

6. Verfahren nach Anspruch 5, wobei die bakteriologische Kontrolle im Falle von Vorhandensein von Escheria coli-Bakterien oder von gesamtkoliformen Bakterien negativ, und ansonsten positiv ist.

## Claims

1. A greywater treatment system comprising: a phyto-purification station (6) receiving greywater and delivering purified water; first and second tanks (9, 11) selectively supplied with purified water derived from the phyto-purification station (6) via a three-way solenoid valve (T), and two solenoid valves (9a, 11a) for selectively emptying their contents towards a sewage network (A); a main tank (12) supplied by the first and second tanks (9, 11) respectively via first and second solenoid valves (9c, 11c); a bacteriological monitoring apparatus (14) connected to the first and second tanks (9, 11) for selectively carrying out for the first or second tanks (9, 11) a bacteriological analysis control requiring a predetermined time for establishment thereof; and a controller controlling the bacteriological monitoring apparatus (14) and the solenoid valves (T, 9c, 11c, 9a, 11a) to cyclically control the sequence of following operations:
- controlling the three-way solenoid valve (T) so that it supplies the first tank (9) and triggering the establishment of a bacteriological control of the purified water contained in the second tank (11);
- upon completion of the establishment time of the control, transferring the content of the second tank (11) towards the main tank (12) if the control is favourable or towards the sewage network (A) if the control is unfavourable;
- controlling the three-way solenoid valve (T) so that it supplies the second tank (11) and triggering the establishment of a bacteriological control of the purified water contained in the first tank (9);
- upon completion of the establishment time of the control, transferring the content of the first tank (9) towards the main tank (12) if the control is favourable or towards the sewage network (A) if the control is unfavourable.

2. The system according to claim 1, comprising a solenoid valve for emptying all or part of the content of the main tank (12) towards the sewage network (A).

3. The system according to claim 1 or 2, comprising an additional tank (13) intended to receive an overflow of purified water that cannot be contained in the main tank (12).

4. The system according to one of the preceding claims, including a monitoring apparatus dedicated to the detection of the presence of Escherichia coli bacteria and/or of total coliforms, and wherein the bacteriological control is unfavourable in case of presence of Escherichia coli bacteria or total coliforms, and favourable otherwise.

5. A method of treating greywater in a plant comprising: a phyto-purification station (6) receiving greywater and delivering purified water; first and second tanks (9, 11) selectively supplied with purified water derived from the phyto-purification station (6); a main tank (12) supplied by the first or second tank (9, 11); a bacteriological monitoring apparatus (14) connected to the first and second tanks (9, 11) to selectively carry out for the first or second tank (9, 11) a bacteriological analysis control requiring a predetermined time for establishment thereof; this method comprising the sequence of following cyclically-triggered operations:
- supplying the first tank (9) with the purified water from the phyto-purification station, and triggering the establishment by the apparatus (14) of a bacteriological control of the purified water contained in the second tank (11);
- upon completion of the establishment time of the control, transferring the content of the second tank (11) towards the main tank (12) if the control is favourable or towards a sewage network (A) if the control is unfavourable;
- supplying the second tank (11) with the purified water from the phyto-purification station and triggering the establishment by the apparatus (14) of a bacteriological control of the purified water contained in the first tank (9);
- upon completion of the establishment time of the control, transferring the content of the first tank (9) towards the main tank (12) if the control is favourable or towards the sewage network (A) if the control is unfavourable.

6. The method according to claim 5, wherein the bacteriological control is unfavourable in case of presence of Escherichia coli bacteria or total coliforms, and favourable otherwise.
